Europäisches Patentamt

European Patent Office

Office européen des brevets

(19)

(11) Veröffentlichungsnummer : **0 033 782**
**B1**

(12) **EUROPÄISCHE PATENTSCHRIFT**

(45) Veröffentlichungstag der Patentschrift :
26.10.83

(21) Anmeldenummer : 80108168.8

(22) Anmeldetag : 23.12.80

(51) Int. Cl.³ : **C 07 C 21/10, C 07 C 17/42,
C 09 K 15/04**

(54) **Stabilisiertes Trichloräthylen.**

(30) Priorität : 31.01.80 DE 3003348

(43) Veröffentlichungstag der Anmeldung :
**19.08.81 Patentblatt 81/33**

(45) Bekanntmachung des Hinweises auf die Patenterteilung : **26.10.83 Patentblatt 83/43**

(84) Benannte Vertragsstaaten :
**AT BE CH DE FR GB IT LI LU NL SE**

(56) Entgegenhaltungen :
**DE A 2 164 394**
**DE B 2 803 529**
**US A 2 797 250**

**Chemical Abstracts, Band 74, Nr. 21, 1971 Columbus, Ohio, USA H. UKIHASHI et al. « Stabilization
of trichloroethylene » Seite 364, Abstract Nr.
111537c**

**Chemical Abstracts Band 74, Nr. 23, 1971 Columbus, Ohio, USA H. UKIHASHI et al. « Stabilizing
trichloroethylene » Seite 404, Abstract Nr.
124762x**

(73) Patentinhaber : **DYNAMIT NOBEL AKTIENGESELL-
SCHAFT**
**Patentabteilung Postfach 1209**
**D-5210 Troisdorf, Bez. Köln (DE)**

(72) Erfinder : **Bieding, Robert**
**Drachenfelsstr. 14**
**D-5463 Unkel (DE)**
Erfinder : **Nestler, Heinz, Dr.**
**Rembrandtstr. 73**
**D-5210 Troisdorf-Eschmar (DE)**

## Stabilisiertes Trichloräthylen

Gegenstand der vorliegenden Erfindung ist ein stabilisiertes Trichloräthylen, das gegenüber der Zersetzung von Säuren durch ein besonders wirksames Gemisch aus Aminen und Epoxydverbindungen stabilisiert ist und außerdem noch weitere, an sich bekannte Stabilisatoren enthält.

Trichloräthylen ist ein vielseitig verwendetes Lösungs- und Reinigungsmittel ; typische Einsatzgebiete für diese Zwecke sind Tri-Tauchlacke und Metallreinigungsverfahren.

Während der Lagerung und Anwendung erleidet Trichloräthylen aufgrund seiner chemischen Struktur bei Einwirkung von Sauerstoff, Säuren und Wärme leicht eine Zersetzung, die durch Licht und durch bestimmte Metallsalze katalysiert wird. Um diese störende Erscheinung, die zu großen Schäden an Anlagen und Reinigungsgut und zum Unbrauchbarwerden des Lösungsmittels führen kann, auszuschalten oder zumindestens zu verzögern oder in ihrer Wirkung abzumildern, wird technisch eingesetztes Trichloräthylen sofort nach seiner Herstellung mit Stabilisatoren versetzt, die in die Kette der zur Zersetzung führenden Reaktionen eingreifen. Dabei wird mit steigender Anforderung an das Lösungsmittel — Kaltreinigung, Dampfreinigung, Leichtmetallentfettung — auch die Zusammensetzung der Stabilisatorsysteme komplizierter, da ein einzelner Zusatz in keinem Fall gegen sämtliche äußeren Einflüsse — Säuren, Metallsalze, Sauerstoff, Metalle, Wärme, Licht und Wasser — wirksam sein kann.

Es sind daher in der Vergangenheit zahlreiche Systeme von Stabilisatoren bekanntgeworden, um technisches Trichloräthylen auch für harte Praxisanforderungen geeignet zu machen. So wird in der DE-PS 10 96 711 ein System aus N-Methylpyrrol, Diisobutylen, Äthylacetat, Diisopropylamin und der 2 : 1-Mischung aus 1,2-Epoxybutan und Epichlorhydrin vorgeschlagen. Weiterhin wird in der DE-PS 11 11 479 ein Gemisch aus Aminen und Epoxydverbindungen als Grundstabilisierung genannt, dem noch andere Stabilisatoren, wie z. B. Methylacetat, hinzugefügt werden können. Auch Ketone, ungesättigte Kohlenwasserstoffe oder Alkylphosphate sind als zusätzliche Stabilisatoren bereits bekannt.

Diesen bekannten Stabilisierungsgemischen ist gemeinsam, daß sie eine säureaufnehmende Komponente und eine Komponente enthalten, die die Stabilisierung gegenüber den übrigen, das Trichloräthylen zersetzenden, Faktoren bewirkt.

In den modernen Metallreinigungsanlagen werden an die Stabilisatoren immer höhere Anforderungen gestellt, so daß viele der bekannten Stabilisierungsgemische zwar bei der Kaltreinigung und der Dampfreinigung die an sie gestellten Forderungen erfüllen, jedoch bei der Leichtmetallentfettung, besonders wenn dabei der Chlorkohlenwasserstoff erhitzt wird, bei einer Dauerbehandlung nicht mehr voll zufriedenstellend sind.

Es bestand deshalb die Aufgabe, ein Stabilisatorgemisch für Trichloräthylen aufzufinden, das den verschärften Bedingungen bei der Metallreinigung, besonders bei der Leichtmetallreinigung standhält. Weiterhin soll das Stabilisatorgemisch frei von Epichlorhydrin sein, da z. B. gemäß den Anforderungen der US-Fed. Spec. O-T-634 ein Stabilisator die Toxizität eines Lösungsmittels nicht erhöhen darf ; demzufolge ist die Mitverwendung von Epichlorhydrin in dem Stabilisator aus ökologischen Gründen unerwünscht.

In Erfüllung dieser Aufgabe wurde nun ein gegen Zersetzung stabilisiertes Trichlorethylen gefunden, das unter Mitverwendung von Aminen und Epoxydverbindungen als säureaufnehmende Komponente und weiteren, den Abbau des Chlorkohlenwasserstoffs verzögernden Mitteln aus der Gruppe der Ketone, Ester und ungesättigten Kohlenwasserstoffen mit Siedepunkten zwischen 90 und 140 °C stabilisiert ist, das dadurch gekennzeichnet ist, daß die säureaufnehmende Komponente außer den Aminen ein Gemisch aus

    a) 1,2-Epoxybutan und
    b) Cyclohexenoxyd und/oder Glycidol

enthält und die Amine einen Siedepunkt im Bereich zwischen 84 und 158 °C aufweisen.

Es ist zwar aus der DE-OS 21 64 394 ein Stabilisierungsgemisch für Trichlorethylen bekannt, das aus Glycidol, Diisopropylamin, 1,2-Epoxibutan und 2,6-Ditert.-Butylphenol besteht. Dort wird jedoch die Anwesenheit des Phenolderivats als zwingend angegeben, weil sonst die stabilisierende Wirkung nicht eintreten würde. Die Phenolderivate haben aber den Nachteil, daß sie bei der Destillation des Trichlorethylens im Rückstand verbleiben und sich dort bei einer Dauerbehandlung, wie sie z. B. bei der oben genannten Metallreinigung auftritt, in ungewünschter Weise anreichert. Dieser Nachteil wird durch das erfindungsgemäße Stabilisierungsgemisch aufgehoben.

Das erfindungsgemäße Gemisch aus den Epoxiverbindungen zeigt in dem zur Prüfung der stabilisierenden Wirkung bekannten Oxydationstest bessere Ergebnisse als jede einzelne Verbindung, wenn sie alleine eingesetzt wird. Auch im Gemisch mit Aminen, besonders im Gemisch mit Diisopropylamin und/oder Dipropylamin, zeigt sich dieser unerwartete, synergistische Effekt.

Als Amine können erfindungsgemäß außer Diisopropylamin und Dipropylamin noch Triäthylamin oder Tripropylamin bevorzugt eingesetzt werden. Prinzipiell eignen sich auch andere, als Stabilisatoren bekannte Amine, sofern ihr Siedepunkt in dem gleichen Bereich wie derjenige der oben genannten Amine liegt und ihr $pK_b$-Wert die gleiche Größenordnung besitzt wie die oben genannten Amine. Als Beispiel seien Äthylendiamin, die Diaminopropane, Tetramethylendiamin und Diallylamin genannt.

Zu den weiteren, an sich bekannten, Stabilisatorzusätzen zählen Ketone, Ester und ungesättigte Kohlenwasserstoffe mit Siedepunkten zwischen 90 und 140 °C. Als besonders wirksame Vertreter seien genannt : Methyläthylketon, Methyl- und Äthylester niederer $C_1$-$C_4$-Carbonsäuren, (z. B. Äthylacetat) Isopren, Cyclohexen, Diisobutylen oder Propylentrimere.

Die Menge dieser zusätzlichen Stabilisatoren in dem Trichloräthylen soll zwischen 0,2 und 5 Gew.-% liegen. Größere Mengen sind prinzipiell auch möglich, erhöhen aber nicht die stabilisierende Wirkung.

Die zuzusetzenden Mengen an Aminen und Epoxiden richten sich nach den Anforderungen der praktischen Anwendung, bei der eine bestimmte Alkalität und ein bestimmtes Säureaufnahmevermögen verlangt werden. Für die beanspruchte Stabilisatorenzusammensetzung sind unter diesem Gesichtspunkt folgende Mengenbereiche wirksam : Amine zwischen 50 und 150 ppm, bevorzugt 80 bis 120 ppm und das Epoxydgemisch aus 1,2-Epoxybutan und Glycidol und/oder Cyclohexenoxyd mit einem Epoxydsauerstoff-Äquivalent im Bereich von 0,02 bis 0,1 Gew.-%.

Diesen Epoxydsauerstoffäquivalenten, die auf NaOH umgerechnet den Bereich von 0,05 bis 0,25 Gew.-% umfassen, entsprechen bei alleinigem Einsatz von 1,2-Epoxybutan 900 bis 4 500 ppm, von Glycidol 925 bis 4 600 ppm und von Cyclohexenoxyd 1 225 bis 6 200 ppm. Wegen der Mischung von 1,2-Epoxybutan mit mindestens einem der anderen Epoxyde werden die angegebenen Grenzwerte der Einzelkomponenten nicht erreicht ; die untere Grenze wird z. B. durch das Gemisch 450 ppm Epoxybutan und 465 ppm Glycidol, die obere durch das Gemisch 3 000 ppm Epoxybutan und 2 070 ppm Cyclohexenoxyd charakterisiert. Zusatzmengen, die unterhalb der angegebenen Untergrenze liegen, bieten keine Gewähr für eine sichere Langzeitwirkung ; Zusatzmengen, die die obere Grenze wesentlich überschreiten, bringen bei nur noch geringem Stabilitätszuwachs eine unnötig hohe ökonomische Belastung.

Zur Prüfung der stabilisierenden Wirkung werden die stabilisierten Trichloräthylen-Proben einem Test in Anlehnung an die USA-Federal-Specification O-T-634 b unterzogen. Diese, im folgenden als Oxidationstest bezeichnete Prüfung, wird folgendermaßen durchgeführt :

Es werden je 200 ml des stabilisierten Trichloräthylens und ein Metallteststreifen (Eisen) in einen Erlenmeyerkolben mit zwei seitlich angesetzten Stutzen gegeben. Ein weiterer Fe-Testmetallstreifen befindet sich in der Dampfzone des Lösungsmittels. Das Perchloräthylen wird mittels einer 150-Watt-Lampe aufgeheizt, verdampft und in einem Kühler kondensiert. Der Rückfluß des Kondensats erfolgt dabei über den in der Dampfzone befindlichen Teststreifen. Außerdem wird über ein Dosierventil (ca. 12 Blasen/Min.) wassergesättigter Sauerstoff (aus einem senkrecht stehenden, in Wasser eintauchenden Glasrohr mit 5 mm Durchmesser) in das Lösungsmittel geleitet. Die Versuchsdauer beträgt 48 Stunden.

Der Versuch wird analog mit Teststreifen aus Zink und Aluminium durchgeführt und, als weitere Verschärfung der Bedingungen, auch in Gegenwart von 0,1 % Wasser im Lösungsmittel.

Zur Beurteilung der Qualität des untersuchten stabilisierten Trichloräthylens werden die folgenden Kriterien herangezogen :

1. Aussehen der Metallblättchen in Flüssigkeit und Dampfraum : + = völlig unverändert, − = geringe, aber deutlich sichtbare Korrosion, − − = starke Korrosion.

Treten hierbei auch nur in einem Falle Korrosionserscheinungen auf, ist das Lösungsmittel nicht universell einsetzbar.

2. Aufrechterhaltung eines pH-Wertes oberhalb pH 7. Die Bestimmung des pH-Wertes erfolgt nach Ausschütteln des Lösungsmittels mit derselben Menge neutral gestellten Wassers in der wässrigen Phase. Beim Erhalt von pH-Werten von 7 und kleiner als 7 muß das Stabilisatorgemisch als ungeeignet angesehen werden.

3. Aufrechterhaltung eines gewissen Säureaufnahmevermögens (SAV). Zur Ermittlung dieses Säureaufnahmevermögens werden die Proben nach folgender, unter Heranziehung der ASTM D 2942-74 festgelegter Methode untersucht.

A) Jeweils 10 ml des zu untersuchenden Lösungsmittels werden in einem 250 ml Erlenmeyerkolben mit genau 25 ml eines Hydrochlorierungs-Reagens (bestehend aus 3,4 ml 37 %iger Salzsäure in 400 g über Kalziumchlorid getrocknetem Isopropanol) versetzt, durch Umschwenken gleichmäßig vermischt und nach einer Reaktionszeit von 5 Minuten und Zugabe von 5 Tropfen einer Bromthymolblau-Indikatorlösung (Herstellung : 0,1 g Bromthymolblau, gelöst in 1,6 ml 0,1 n Natronlauge und mit Äthanol auf 100 ml aufgefüllt) mit 0,1 n alkoholischer KOH auf einen bleibenden blauen Umschlagpunkt titriert.

B) In einem Blindversuch werden 25 ml des Hydrochlorierungs-Reagens nach Zugabe von 5 Tropfen Bromthymolblau-Indikatorlösung ebenfalls mit 0,1 n alkoholischer KOH auf blauen Endpunkt titriert.

Berechnung : (B − A) × 0,4/Volumen der Probe (ml) × Dichte der Probe (g/ml) = SAV
(SAV als Gew.-% NaOH)

A = Verbrauch in ml 0,1 n KOH bei der Untersuchung des Trichloräthylens,

B = Verbrauch in ml 0,1 n KOH im Blindversuch.

Wenn das Trichloräthylen nach dem Oxidationstest kein Säureaufnahmevermögen mehr besitzt, ist es ungenügend stabilisiert.

### Beispiel 1 (Zum Vergleich)

In der Tabelle I sind die Ergebnisse von Oxidationstest-Versuchen mit Trichloräthylen wiedergegeben, das nach dem Stand der Technik mit einer Mischung von 1,2-Epoxybutan/Epichlorhydrin bzw. mit nur einem Epoxyd im Stabilisatorgemisch stabilisiert wurde. Die angegebenen Zahlenwerte für die einzelnen Stabilisatoren geben die Menge dieser Verbindungen in dem damit stabilisierten Trichloräthylen in ppm an.

Tabelle I

| Versuchs-Nr. | I | | II | | III | | IV | |
|---|---|---|---|---|---|---|---|---|
| Diisopropylamin | 100 | | 100 | | 100 | | 100 | |
| Epichlorhydrin | 1 000 | | — | | — | | — | |
| 1,2-Epoxybutan | 2 000 | | 2 800 | | — | | — | |
| Cyclohexenoxyd | — | | — | | 5 000 | | — | |
| Glycidol | — | | — | | — | | 3 700 | |
| Methyläthylketon | — | | 2 500 | | 2 000 | | 2 500 | |
| Diisobutylen | 2 000 | | 2 500 | | 2 500 | | 2 500 | |
| N-Methylpyrrol | 200 | | — | | — | | — | |
| Äthylacetat | 2 000 | | — | | — | | — | |
| Metall (+ 0,1 % H$_2$O) | Fe | Zn | Fe | Zn | Fe | Zn | Fe | Zn |
| Blättchen in Flüss. | – | – | – | + | + | + | + | + |
| Blättchen in Dampf. | – – | – – | – – | + | – | + | – | – |
| pH-Wert | 10,1 | 9,9 | 1,8 | 7,6 | 2,0 | 8,2 | 10,0 | 10,0 |
| SAV (Gew.-% NaOH) | 0,09 | 0,08 | kein | 0,03 | kein | 0,28 | 0,21 | 0,22 |

Beispiel 2

Trichloräthylen wurde mit den in Tabelle II genannten Mengen (in ppm angegeben) der erfindungsgemäßen Gemische aus 1,2-Epoxybutan/Cycohexenoxid versetzt. Das so stabilisierte Gemisch wurde dem Oxidationstest unterworfen ; die Ergebnisse sind in der Tabelle II aufgeführt. Die Ansätze mit Eisen und Zink wurden zusätzlich mit 0,1 % Wasser versetzt ; die mit Aluminium wasserfrei durchgeführt.

Tabelle II

| Versuchs-Nr. | V | | | VI | | |
|---|---|---|---|---|---|---|
| Diisopropylamin | 100 | | | — | | |
| Dipropylamin | — | | | 80 | | |
| 1,2-Epoxybutan | 2 800 | | | 2 500 | | |
| Cyclohexenoxyd | 1 100 | | | 1 200 | | |
| Methyläthylketon | 2 500 | | | 2 000 | | |
| Diisobutylen | 2 500 | | | 3 000 | | |
| Metall | Fe | Zn | Al | Fe | Zn | Al |
| Blättchen in Flüss. | + | + | + | + | +· | + |
| Blättchen in Dampf | + | + | + | + | + | + |
| pH-Wert | 8,4 | 8,0 | 8,1 | 8,1 | 8,0 | 8,0 |
| SAV (Gew.-% NaOH) | 0,07 | 0,11 | 0,10 | 0,06 | 0,09 | 0,09 |

Beispiel 3

Trichloräthylen wurde mit den in Tabelle III angegebenen ppm-Mengen der dort genannten Stabilisatoren versetzt. Anschließend wurde der Oxidationstest durchgeführt, dessen Ergebnis in Tabelle III zusammengefaßt ist.

(Siehe Tabelle III Seite 5 f.)

Tabelle III

| Versuchs-Nr. | VII | | | VIII | | | IX | | |
|---|---|---|---|---|---|---|---|---|---|
| Diisopropylamin | 80 | | | — | | | 100 | | |
| Dipropylamin | — | | | 100 | | | — | | |
| 1,2-Epoxybutan | 2 800 | | | 3 300 | | | 3 000 | | |
| Glycidol | 880 | | | 400 | | | 500 | | |
| Methyläthylketon | 2 500 | | | — | | | 2 500 | | |
| Diisobutylen | 2 500 | | | 2 500 | | | — | | |
| Äthylacetat | — | | | 2 500 | | | — | | |
| Metall | Fe | Zn | Al | Fe | Zn | Al | Fe | Zn | Al |
| Blättchen in Flüss. | + | + | + | + | + | + | + | + | + |
| Blättchen in Dampf | + | + | + | + | + | + | + | + | + |
| pH-Wert | 10,3 | 10,1 | 10,2 | 10,1 | 10,2 | 10,2 | 10,2 | 10,2 | 10,2 |
| SAV (Gew.-% NaOH) | 0,06 | 0,09 | 0,09 | 0,06 | 0,06 | 0,12 | 0,06 | 0,04 | 0,06 |
| Ausgangswerte der eingesetzten Proben : | | | | | | | | | |
| pH-Wert | 10,3 | | | 10,2 | | | 10,2 | | |
| SAV | 0,21 | | | 0,21 | | | 0,22 | | |

Beispiel 4

Ein Trichloräthylenansatz mit der Stabilisierung X
   100 ppm Diisopropylamin
3 300 ppm 1,2-Epoxybutan
   400 ppm Glycidol
2 500 ppm Methyläthylketon
2 500 ppm Diisobutylen

wurde 5 mal hintereinander in einer Glasapparatur aus Kolben, Liebigkühler und Vorlage überdestilliert und anschließend dem Oxidationstest mit Eisen- und Zinkblättchen in Gegenwart von 0,1 % Wasser unterworfen. In keinem Fall zeigten die Teststreifen Korrosionserscheinungen, die Lösungsmittel waren in Ordnung.

Beispiel 5

Zwei Proben von Trichloräthylen, die die in der folgenden Tabelle genannten Stabilisatoren (Stabilisatormenge in ppm angegeben) enthielten, wurden einer speziellen Prüfung auf Eignung als Entfettungsmittel von Aluminium unterworfen.

| Versuchs-Nr. | XI | XII |
|---|---|---|
| Diisopropylamin | 100 | 100 |
| 1,2-Epoxybutan | 2 800 | 3 100 |
| Glycidol | 880 | 580 |
| Methyläthylketon | 2 500 | 2 500 |
| Diisobutylen | 2 500 | 2 500 |

Die Ansätze wurden durch einfache Destillation in drei gleich große Fraktionen zerlegt. Dann wurden aus jeder Fraktion 100 ml mit 100 ml Toluol, 18 g Aluminiumflitter und 0,7 g Aluminiumchlorid versetzt und 18 Stunden in einem Kolben mit aufgesetztem Rückflußkühler bei einer Ölbadtemperatur von 125 °C gekocht (2 mal 9 Stunden mit zwischenzeitlichem Abkühlen im erkaltenden Ölbad). Sämtliche 6 Einzeltests verliefen ohne heftige exotherme Reaktion.

**Ansprüche** (für die Vertragsstaaten : BE, CH, DE, FR, GB, IT, LI, LU, NL, SE)

1. Gegen Zersetzung stabilisiertes Trichlorethylen, das unter Mitverwendung von Aminen und Epoxyd-Verbindungen als säureaufnehmende Komponente und weiteren, den Abbau des Chlorkohlenwasserstoffs verzögernden Mitteln aus der Gruppe der Ketone, Ester und ungesättigten Kohlenwasserstoffe mit Siedepunkten zwischen 90 und 140 °C stabilisiert ist, dadurch gekennzeichnet, daß die säureaufnehmende Komponente außer den Aminen ein Gemisch aus

a) 1,2 Epoxibutan und
b) Cyclohexenoxyd und/oder Glycidol

enthält und die Amine einen Siedepunkt im Bereich zwischen 84 und 158 °C aufweisen.

2. Stabilisiertes Trichlorethylen gemäß Anspruch 1, dadurch gekennzeichnet, daß es als Amin Diisopropylamin und/oder Dipropylamin enthält.

3. Stabilisiertes Trichlorethylen gemäß Anspruch 1 oder 2, dadurch gekennzeichnet, daß das Epoxydgemisch mengenmäßig einem Epoxydsauerstoffäquivalent von 0,02 bis 0,1 Gew.-% entspricht.

4. Stabilisiertes Trichlorethylen gemäß einem der Ansprüche 1 bis 3, dadurch gekennzeichnet, daß die Minimalmengen an Epoxyden sich anteilig aus den der unteren Grenze ensprechenden Mengen von 900 ppm 1,2-Epoxibutan, 1 225 ppm Cyclohexenoxyd und 925 ppm Glycidol, und die Maximalmengen an Epoxyden sich anteilig aus den der oberen Grenze entsprechenden Mengen von 4 500 ppm 1,2-Epoxibutan, 6 200 ppm Cyclohexenoxyd und 4 600 ppm Glycidol ergeben.

**Ansprüche** (für den Vertragsstaat AT)

1. Verfahren zur Verhinderung der Zersetzung von Trichlorethylen durch Untermischen von Aminen und Epoxydverbindungen als säureaufnehmende Komponente und weiteren, den Abbau des Chlorkohlenwasserstoffs verzögernden Mitteln aus der Gruppe der Ketone, Ester und ungesättigten Kohlenwasserstoffe mit Siedepunkten zwischen 90 und 140 °C in das Trichlorethylen, dadurch gekennzeichnet, daß man dem Trichlorethylen als säureaufnehmende Komponente außer Aminen mit einem Siedepunkt im Bereich zwischen 84 und 158 °C ein Gemisch aus

a) 1,2 Epoxibutan und
b) Cyclohexenoxid und/oder Glycidol

untermischt.

2. Verfahren gemäß Anspruch 1, dadurch gekennzeichnet, daß die Menge des unterzumischenden Epoxydgemischs einem Epoxydsauerstoffäquivalent von 0,02 bis 0,1 Gew.-% entspricht.

3. Verfahren gemäß Ansprüchen 1 und 2, dadurch gekennzeichnet, daß die Minimalmengen an Epoxyden sich anteilig aus den der unteren Grenze entsprechenden Mengen von 900 ppm 1,2-Epoxibutan, 1 225 ppm Cyclohexenoxyd und 925 ppm Glycidol, und die Maximalmengen an Epoxyden sich anteilig aus den der oberen Grenze entsprechenden Mengen von 4 500 ppm 1,2-Epoxibutan, 6 200 ppm Cyclohexenoxyd und 4 600 ppm Glycidol ergeben.

**Claims** (for the Contracting States : BE, CH, DE, FR, GB, IT, LI, LU, NL, SE)

1. Trichloroethylene stabilised against decomposition, which is stabilised by concurrent use of amines and epoxy compounds as acid-accepting component and furthermore means retarding the decomposition of the chlorohydrocarbon from the group of ketones, esters and unsaturated hydrocarbons, with boiling points between 90 and 140 °C, characterised in that the acid-accepting component consists of, in addition to the amines a mixture of

a) 1,2-epoxybutane and
b) cyclohexeneoxide and/or glycidol

and the amine possesses a boiling point in the region between 84 and 158 °C.

2. Stabilised trichloroethylene according to claim 1, characterised in that it contains, as amine, diisopropylamine and/or dipropylamine.

3. Stabilised trichloroethylene according to claim 1 or 2, characterised in that the epoxide mixture corresponds quantitatively to an epoxide oxygen equivalent of 0.02 to 0.1 % by weight.

4. Stabilised trichloroethylene according to one of claims 1 to 3, characterised in that the minimum allowance of epoxides result proportionately from the amount of 900 ppm 1,2-epoxybutane, 1 225 ppm cyclohexeneoxide and 925 ppm glycidol corresponding to the lower limit and the maximum amounts of epoxides result proportionately from the amounts of 4 500 ppm 1,2-epoxybutane, 6 200 ppm cyclohexeneoxide and 4 600 ppm glycidol corresponding to the upper limit.

**0 033 782**

**Claims** (for the Contracting State AT)

1. Process for stabilising trichloroethylene against decomposition by mixing the trichloroethylene with amines and epoxy compounds as acid-accepting component and furthermore means retarding the decomposition of the chlorohydrocarbon from the group of ketones, esters and unsaturated hydrocarbons, with boiling points between 90 and 140 °C, characterized in that as acid-accepting component, in addition to amines, possessing a boiling point in the region between 84 and 158 °C, a mixture of

a) 1,2-epoxybutane and
b) cyclohexeneoxide and/or glycidol

is intermixed with the trichloroethylene.

2. Process according to claim 1, characterised in that the epoxide mixture corresponds qualitatively to an epoxide oxygen equivalent of 0.02 to 0.1 % by weight.

3. Process according to claim 1 or 2, characterised in that the minimum allowance of epoxides results proportionately from the amounts of 900 ppm 1,2-epoxybutane, 1 225 ppm cyclohexeneoxide and 925 ppm glycidol corresponding to the lower limit and the maximum amounts of epoxides result proportionately from the amounts of 4 500 ppm 1,2-epoxybutane, 6 200 ppm cyclohexeneoxide and 4 600 ppm glycidol corresponding to the upper limit.


**Revendications** (pour les Etats Contractants : BE, CH, DE, FR, GB, IT, LI, LU, NL, SE)

1. Trichloréthylène stabilisé à l'encontre d'une décomposition qui est stabilisé par l'incorporation d'amines et de composés époxydes à titre de constituant de fixation d'acides et par l'incorporation d'autres agents retardant la décomposition de l'hydrocarbure chloré, choisis dans le groupe des cétones, des esters et des hydrocarbures insaturés dont le point d'ébullition se situe entre 90° et 140 °C, caractérisé en ce que le constituant de fixation des acides contient, en plus des amines, un mélange de

a) epoxy-1,2 butane et
b) oxyde de cyclohexène et/ou glycidol

et les amines présentent un point d'ébullition se situant dans l'intervalle compris entre 84° et 158 °C.

2. Trichloréthylène stabilisé selon la revendication 1, caractérisé en ce qu'il contient comme amine la diisopropylamine et/ou la dipropylamine.

3. Trichloréthylène stabilisé selon la revendication 1 ou 2, caractérisé en ce que le mélange des époxydes correspond, en quantité, à un équivalent d'oxygène d'époxyde de 0,02 à 0,1 % en poids.

4. Trichloréthylène stabilisé selon l'une des revendications 1 à 3, caractérisé en ce que les quantités minimales d'époxydes ressortent proportionnellement des quantités de 900 ppm d'époxy-1,2 butane, 1 225 ppm d'oxyde de cyclohexène et 925 ppm de glycidol pour la limite inférieure, et les quantités maximales d'époxydes ressortent proportionnellement des quantités de 4 500 ppm d'époxy-1,2 butane, 6 200 ppm d'oxyde de cyclohexène et 4 600 ppm de glycidol pour la limite supérieure.


**Revendications** (pour l'Etat Contractant AT)

1. Procédé de stabiliser trichloréthylène à l'encontre d'une décomposition par incorporation d'amines et de composés époxydes à titre de constituant de fixation d'acides et par l'incorporation d'autres agents retardant la décomposition de l'hydrocarbure chloré, choisis dans le groupe des cétones, des esters et des hydrocarbures insaturés dont le point d'ébullition se situe entre 90° et 140 °C, caractérisé en ce que comme constituant de fixation des acides contient, en plus des amines, présentant un point d'ébullition se situant dans l'intervalle compris entre 84° et 158 °C, on incorpore un mélange de

a) epoxy-1,2 butane et
b) oxyde de cyclohexène et/ou glycidol.

2. Procédé selon la revendication 1, caractérisé en ce que 1 mélange des époxydes correspond, en quantité, a un équivalent d'oxygène d'époxyde de 0,02 à 0,01 % en poids.

3. Procédé selon l'une des revendications 1 ou 2, caractérisé en ce que les quantités minimales d'époxydes ressortent proportionnellement des quantités de 900 ppm d'époxy-1,2 butane, 1 225 ppm d'oxyde de cyclohexène et 925 ppm de glycidol pour la limite inférieure, et les quantités maximales d'époxydes ressortent proportionnellement des quantités de 4 500 ppm d'époxy-1,2 butane, 6 200 ppm d'oxyde de cyclohexène et 4 600 ppm de glycidol pour la limite supérieure.